# EUROPEAN PATENT SPECIFICATION

(11) **EP 1 516 540 B1**
(45) Date of publication and mention of the grant of the patent: **26.09.2012**
(21) Application number: 04019637.0
(22) Date of filing: 19.08.2004
(51) Int. Cl.: A23K 1/16, A23K 1/18

(54) **Use of vitamin D compounds**
Verwendung von Vitamin D Verbindungen
Utilisation de composés de la vitamine D

(30) Priority: 22.09.2003 EP 03021362
(43) Date of publication of application: 23.03.2005
(73) Proprietor: DSM IP Assets B.V., 6411 TE Heerlen (NL)
(72) Inventor: Simoes-Nunes, Carlos, F-68128 Village-Neuf (FR); Weber, Gilbert, M., CH-4312 Magden (CH)
(74) Representative: Schwander, Kuno

(56) References cited:
- WO-A-90/09179
- WO-A-03/059358
- US-A- 5 154 925
- US-A- 5 695 794
- DATABASE BIOSIS [Online] BIOSCIENCES INFORMATION SERVICE, PHILADELPHIA, PA, US; 1997, ROBERSON K D: "Effect of 25-hydroxycholecalciferol level on performance and bone strength in weanling pigs" XP002304504 Database accession no. PREV199800063548 & JOURNAL OF ANIMAL SCIENCE, vol. 75, no. SUPPL. 1, 1997, page 14, 89TH ANNUAL MEETING OF THE AMERICAN SOCIETY OF ANIMAL SCIENCE, SOUTHERN SECTION; BIRMINGHAM, ALABAMA, USA; FEBRUARY 1-4, 1997 ISSN: 0021-8812

## Description

The present invention relates to the use of vitamin D compounds for improving bone strength in pigs. More particularly, the invention relates to the use of 25-hydroxy vitamin D₃, in combination with vitamin D₃ , in the manufacture of a food or veterinary composition for improvement of bone strength in pigs. In another aspect, the invention relates to a method of improving bone strength in pigs, which comprises administering to a pig in need of such treatment 25-hydroxy vitamin D₃, in combination with vitamin D₃ . In still another aspect, the invention relates to a pig food or premix therefor, comprising 25-hydroxy vitamin D₃ in combination with vitamin D₃.

In many pig herds locomotor disorders are an important problem. In herds where the value of individual purebred pigs is high or in breeding companies that produce premium hybrid pigs the cost of the mentioned disorders can be high. Osteochondrosis (dyschondroplasia), osteoarthrosis and leg weakness can cause severe crippling, and the frequency of these conditions has been high in pigs with otherwise ideal growth characteristics. These disorders are a worldwide problems that have caused concern in the pig industry for 50 or more years. Thus and accordingly, adequate bone resistance and mineralisation are a permanent objective in swine breeding.

WO 1990/09179 (Univ. Georgia Research Foundation et al.) and US 5,145,925 (Edwards, Jr. et al) teach use of various Vitamin D3 derivatives in poultry to ameliorate tibia dyschondroplasia.

Roberson 1997 J. Animal Science 75 Suppl 13. Abstract 49 used 25-hydroxyvitamin D3 in swine with mixed results.

US 5,695,794 (Stark et al) teaches use of 25-hydroxyvitamin D3 to ameliorate tibia dyschondroplasia in poultry. The formulation may include rice hulls as a comestible diluent.

WO 03/059358 (Roche Vitamins AG) teaches use of a stabilized formulation of 25-hydroxy vitamin D3 in various premixes and feed.

In accordance with the present invention it has been found that the above problems in pig breeding can be eliminated or substantially ameliorated by administering to the animals an effective amount of 25-hydroxy vitamin D₃ in combination with vitamin D₃.

25-hydroxy vitamin D₃ and vitamin D₃ (hereinafter : inventive ingredients) are suitably administered together with the food. The term food as used herein comprises both solid and liquid food as well as drinking fluids such as drinking water. Particularly, inventive ingredients can be added as a formulated powder to a premix containing other minerals, vitamins, amino acids and trace elements which is added to regular pig food and thorough mixing to achieve even distribution therein.

In the manufacture of a pig food in accordance with the invention, from about 10 µg/kg to about 100 µg/kg of 25-hydroxy vitamin D₃ and, if required, from about 200 IU/kg to about 4,000 IU/kg of vitamin D₃ are added to regular pig food. Alternatively, a food premix may be prepared on the basis of regular food components by adding active ingredients to such food components in higher concentration, e.g., in a concentration of from about 2 mg/kg to about 20 mg/kg of 25-hydroxy vitamin D₃ and, if required, from about 40,000 IU/kg to about 800,000 IU/kg of D₃. If 5 kg of such premix are added per 1000 kg of regular food this would typically meet the individual need of the animal by normal food consumption. For piglets from 21 to 60 days of age preferable dosages are in the range of from about 10 to 30 µg/kg of 25-hydroxy vitamin D₃ and from about 500 to about 2,000 IU/kg of vitamin D₃. For pigs older than 60 days preferable dosages are in the range of and from about 10 to about 20 µg of 25-hydroxy vitamin D₃ and from about 300 to about 1,200 IU/kg of vitamin D₃.

The invention is further illustrated by the following examples.

### Example 1

Growing-fattening pigs of an initial body weight of 64.8±5.03 kg were fed a diet based on maize, barley and soybean meal *ad libitum* for 34 days. Groups of 16 animals each were fed as follows:
A : basal diet (unmedicated),
B : basal diet + 1,200 IU/kg vitamin D₃
C : basal diet + 30 µg/kg 25-hydroxy vitamin D₃ (Hy·D® 1.25% Beadlet, Roche Vitamins AG, Basel, Switzerland)
D : basal diet + 600 IU/kg vitamin D₃ + 15 µg/kg 25-hydroxy vitamin D₃ (Hy·D® 1.25% Beadlet, Roche Vitamins AG, Basel, Switzerland).

The effects of the addition of 25-hydroxy vitamin D₃ and vitamin D₃ to the diet were determined by measuring the strength (Newton at the breaking point) of the bones of the animals. The results are shown in Table 1.

**Table 1**

| Variables | A (n=16) | B (n=16) | C (n=16) | D (n=16) |
|---|---|---|---|---|
| Strength - metacarpals | 423±107^{(1)c} | 463±48^{ce} | 520±72^{df} | 619±147^{df} |
| | (100) | (109) | (123) | (146) |
| Strength - metatarsals | 529±102⁽¹⁾ | 553±169 | 545±105 | 612±92 |
| | (100) | (105) | (103) | (116) |
| Strength - metacarpals | 469±102^{(2)a} | 508±113^{ac} | 533±88^{ac} | 616±119^{bd} |
| and metatarsals | (100) | (107) | (112) | (129) |

| | | | | |
|---|---|---|---|---|
| ⁽¹⁾ - mean ± standard deviation of the mean of 8 determinations; (²) - mean ± standard deviation of the mean of 16 determinations; a, b, - Mean values within a row with unlike superscript letters were significantly different: a-bP<0.01;c-d and e -fP<0.05. | | | | |

The addition of 25-hydroxy vitamin D₃ and of the combination of 25-hydroxy vitamin D₃ with vitamin D₃ clearly improved the strength of the bones of the growing-finishing pig. The increase of the bone resistant was particularly high with the combination of 25-hydroxy vitamin D₃ with vitamin D₃.
Better bone quality is associated with a reduction of locomotor disorders.

### Example 2

Weaner piglets of an initial body weight of 8.4±1.2 kg were fed a diet based on maize, barley and soybean meal ad libitum for 33 days. Groups of 31 animals each were fed as follows:
A : basal diet + 1,000 IU/kg vitamin D₃
B : basal diet + 2,000 IU/kg vitamin D₃
C : basal diet + 40 µg/kg 25-hydroxy vitamin D₃ (Hy·D® 1.25% Beadlet, Roche Vitamins AG, Basel, Switzerland)
D : basal diet + 1,000 IU/kg vitamin D₃ + 20 µg/kg 25-hydroxy vitamin D₃ (Hy·D® 1.25% Beadlet, Roche Vitamins AG, Basel, Switzerland).

The effects of the addition of 25-hydroxy vitamin D₃ and vitamin D₃ to the diet were determined by measuring the strength (Newton at the breaking point) of the bones of the animals. The results are shown in Table 2.

**Table 2**

| Variables | A (n= 31) | B (n= 31) | C (n= 31) | D (n= 31) |
|---|---|---|---|---|
| Strength - femur | 802±126^{(1)a} | 873±123^{ab} | 803±82^{a} | 929±63^{b} |
| | (100) | (109) | (100) | (116) |

| | | | | |
|---|---|---|---|---|
| ⁽¹⁾ - mean ± standard deviation of the mean of 8 determinations; a, b, - mean values within a row with unlike superscript letters were significantly different: a - b P < 0.05. | | | | |

In this example the increase of the bone resistant was particularly high with the combination of 25-hydroxy vitamin D₃ with vitamin D₃.

### Example 3

A pig food containing 25-hydroxy vitamin D₃ and vitamin D₃ can be prepared as follows:

| Ingredients | [%] |
|---|---|
| Soybean meal | 18 |
| Maize | 53 |
| Barley | 14 |
| Oat meal | 6 |
| Wheat bran | 5.4 |
| Soy oil | 1 |
| Minerals | 1.5 |
| Synthetic amino acids premix | 0.5 |
| Vitamins and trace elements premix | 0.6 |

(containing from about 1.25 g to 5 g of Hy·D® 1.25% Beadlet per 100 g of premix) The ingredients are mixed together and if needed the obtained mash food can be pelleted.

### Example 4

A premix for a piglet food containing 25-hydroxy vitamin D₃ and vitamin D₃ can be prepared as follows:

| Ingredients | [%] |
|---|---|
| Hy·D® 1.25% Beadlet | 0.0320 |
| Vitamin A 500 | 0.8000 |
| Vitamin E 50% | 8.0000 |
| Vitamin D3 500 | 0.0800 |
| Vitamin K3 100% MSB / 51% | 0.0800 |
| Vitamin B1 98% | 0.0714 |
| Vitamin B2 80% | 0.1750 |
| Vitamin B6 99% | 0.1212 |
| Vitamin B 12 0.1 % | 1.0000 |
| Biotin 2% | 0.2000 |
| Folic Acid 80% | 0.0227 |
| Niacin 99.5% | 0.7035 |
| Calpan 98% | 0.4082 |
| Vitamin C | 4.0000 |
| Cholinchloride 60% | 12.0000 |
| Copper sulfate 25% | 12.8000 |
| Iron sulfate 30% | 10.0000 |
| Mangan oxide 62% | 1.6129 |
| Zinc oxide 76% | 5.2632 |
| Cobalt carbonate 5% | 0.0600 |
| Calcium jodate 62% | 0.0323 |
| Sodium selenite 1%/BMP | 0.8001 |
| BHT 100% | 2.0000 |
| Carrier Combination | 6.0000 |
| LACANTES S36400-Z | 10.0000 |
| Limestone | 23.7375 |

All ingredients are carefully mixed together and 0.5% (5kg/1000 kg of food) of this premix is added to the final piglet food.

Alternatively, 25-hydrox vitamin D3 can also be added in a 1% diluted premix, containing a suitable carrier. Such carrier can be wheat flour, wheat middlings, corn cobbs, rice hulls, almond shells or calcium carbonate alone or in variable mixtures of several of these carriers. A typical formula is:

| Ingredients | [%] |
|---|---|
| Wheat flour | 80.00 |
| Calcium carbonate | 19.68 |
| Hy·D® 1.25% Beadlet | 0.32 |

All ingredients are carefully mixed together and 0.05% (0.5 kg/1000 kg of food) of this premix is added to the final piglet food.

## Claims

1. The use of 25-hydroxy vitamin D₃ in combination with vitamin D₃ , in the manufacture of a food or veterinary composition for improvement of bone strength in pigs.

2. The use as in claim 1 in the manufacture of a pig food comprising from 10 mg/kg to 100 mg/kg of 25-hydroxy vitamin D₃ and from 200 IU/kg to 4,000 IU/kg of vitamin D₃.

3. A food premix for pig feed comprising from 2 mg/kg to 20 mg/kg of 25-hydroxy vitamin D₃ and from 40,000 IU/kg to 800,000 IU/kg of D₃.

## Patentansprüche

1. Verwendung von 25-Hydroxy-Vitamin D₃ in Kombination mit Vitamin D₃ in der Herstellung einer Futtermittelzusammensetzung bzw. einer tierärztlichen Zusammensetzung für die Verbesserung der Knochenfestigkeit beim Schwein.

2. Verwendung nach Anspruch 1 in der Herstellung eines Schweinefuttermittels, das 10 mg/kg bis 100 mg/kg 25-Hydroxy-Vitamin D₃ und 200 IU/kg bis 4000 IU/kg Vitamin D₃ umfasst.

3. Futtermittelvormischung für ein Schweinefutter, das 2 mg/kg bis 20 mg/kg 25-Hydroxy-Vitamin D₃ und 40.000 IU/kg bis 800.000 IU/kg D₃ umfasst.

## Revendications

1. Utilisation de 25-hydroxyvitamine D₃ en combinaison avec de la vitamine D₃, dans la fabrication d'une composition alimentaire ou vétérinaire destinée à améliorer la solidité des os chez le porc.

2. Utilisation selon la revendication 1, dans la fabrication d'un aliment pour porcs comprenant de 10 mg/kg à 100 mg/kg de 25-hydroxyvitamine D₃ et de 200 UI/kg à 4 000 UI/kg de vitamine D₃.

3. Prémélange d'aliment pour l'alimentation des porcs comprenant de 2 mg/kg à 20 mg/kg de 25-hydroxyvitamine D₃ et de 40 000 UI/kg à 800 000 UI/kg de D₃.
